# EUROPEAN PATENT SPECIFICATION

(11) **EP 3 028 709 B1**
(45) Date of publication and mention of the grant of the patent: **28.08.2019**
(21) Application number: 15201219.1
(22) Date of filing: 23.02.2011
(51) Int. Cl.: A61P 3/02, A61K 31/714, A61K 45/06, A61K 31/155, A61K 31/519, A61K 31/609, A61K 9/00

(54) **ORAL B12 THERAPY**
ORALE B12-THERAPIE
THÉRAPIE B12 ORALE

(30) Priority: 24.02.2010 US 307836 P
(43) Date of publication of application: 08.06.2016
(62) Divisional of application: 11747966.7
(73) Proprietor: Emisphere Technologies, Inc, Cedar Knolls, NJ 07927 (US)
(72) Inventor: CASTELLI, Cristina, Cedar Knolls,New Jersey NJ New Jersey 07927 (US)
(74) Representative: Pons

(56) References cited:
- US-A1- 2010 016 255
- HART N J: "Eligen(R) vitamin B12 & the SNAC carrier for oral delivery", DRUG DELIVERY TECHNOLOGY, DRUG DELIVERY TECHNOLOGY, US, vol. 9, no. 9, October 2009 (2009-10), pages 28-34, XP009170295, ISSN: 1537-2898
- CASTELLI M CRISTINA ET AL: "SNAC co-formulation produces significant enhancement of oral vitaminB12 bioavailability in rats", FASEB JOURNAL, FED. OF AMERICAN SOC. FOR EXPERIMENTAL BIOLOGY, US, vol. 22, no. Meeting Abstract Supplement, April 2008 (2008-04), XP009170259, ISSN: 0892-6638

## Description

This application claims the benefit of U.S. Provisional Application No. 61/307,836, filed February 24, 2010.

### FIELD OF THE INVENTION

This disclosure relates generally to methods of normalizing vitamin B₁₂ levels in patients with low vitamin B₁₂ and to methods of normalizing intersubject variability in the treatment of such patients. This disclosure also relates to methods of reducing methyl malonic acid (MMA) and/or homocysteine levels, and pharmaceutical compositions useful to effect such changes.

### BACKGROUND OF THE INVENTION

Vitamin B₁₂ is important for the normal functioning of the brain and nervous system and for the formation of blood. It is involved in the metabolism of every cell of the body, especially affecting DNA synthesis and regulation but also fatty acid synthesis and energy production. Its effects are still not completely known.

Cyanocobalamin is a stable and widely used form of vitamin B₁₂. Vitamin B₁₂ is excreted in the bile and undergoes some enterohepatic recycling. Absorbed vitamin B₁₂ is transported via specific B₁₂ binding proteins, transcobalamin I and II, to the various tissues. The liver is the main organ for vitamin B₁₂ storage.

Vitamin B₁₂ deficiency occurs from dietary insufficiency, Intrinsic Factor deficiency, intestinal disturbances, such as malabsorption, gastrectomy, gastric atrophy, ileal resection and chronic inflammatory bowel disease. Chronic use of drugs such as proton pump inhibitors and metformin also induce vitamin B₁₂ deficiency. Vitamin B₁₂ deficiency can potentially cause severe and irreversible damage, especially to the brain and nervous system.

The traditional treatment for B₁₂ deficiency is intramuscular (IM) injections. Usually, in the United States injections of cyanocobalamin are given frequently within in the first month (for instance, 6 to 9 injections) followed by maintenance injections as prescribed by the physician. After the initial treatment period or once clinical remission occurs, treatment is continued for life, often on a monthly schedule. Various treatment schedules and doses are described in the literature commencing with frequent dosing and transitioning to less frequent maintenance dosing. *See* Hvas et al., Haematologica 2006, 137:2481-84.

Oral cyanocobalamin has also been used to treat vitamin B₁₂ deficiency, but with many patients failing to respond. One study was in pernicious anemia patients and two were in patients with food cobalamin malabsorption. The results indicated that 80-90% of patients achieved normal serum B₁₂ levels in the 3 month studies and 95% were normalized in the 2.5 year study. Clinical improvements were reported in only 20-30% of the patients (Andres et al., Eur J Intern Med 2003, 18:221-26). A short-term study of 1 week duration with 1000 µg/day oral B₁₂ indicated that B₁₂ levels increased by a mean value of 0.23 µg/L (230 pg/ml) in 17 of 20 elderly patients (Kaltenbach et al., Ann Med Interne 2003, 154:91-95).

Additionally, the ability of oral treatments to match parenteral B₁₂ in rapidly and reliably restoring or maintaining B₁₂ stores in most or all patients is still in question. As a result, IM treatment remains in wide clinical use in some regions, including the United States. A recent systematic review of randomized controlled oral B₁₂ intervention studies examined the biomarker responses to intervention with oral B₁₂ in 8 studies (Hoey et al., Am J Clin Nutr 2009, 89 (suppl):1981S-96S). Cobalamin, MMA, homocysteine (HC) and holotranscobalamin responses were evaluated. It was found that the B₁₂ intakes produced highly variable effects on blood B₁₂ concentrations and that gender or age subgroup analysis failed to account for the variability. The authors noted that some papers failed to report sufficient information to impart a clear picture of the clinical response. Carmel (Food Nutr Bull 2008, 29:S177-S187) also commented on B₁₂ intervention studies, noting that reliance on mean values and group statistics in B₁₂ supplementation studies can often hide subsets of persons with little or no improvement and that in three prospective studies of B₁₂ supplementation in the elderly cited in this paper poor responders were not usually studied further to examine the cause of poor responsiveness. US2010/0016255 A1 discloses oral pharmaceutical compositions (i.e. tablets) comprising (i) SNAC and (ii) vitamin B12 for treatment of vitamin B12 deficiency conditions.

IM B₁₂ administration is inconvenient, relatively costly when medical personnel are involved in dosing, difficult in the frail and elderly where muscle mass is insufficient and may be painful (Butler et al., Fam Pract 2006, 23:279-85). Current oral B₁₂ treatment is still seen as less reliable than parenteral administration and patient monitoring is advised more frequently than with parenteral administration (Lane et al., Ann Pharmacother 2002; 36: 1268-72). Thus, there is a continuing need for more reliable and effective oral treatments for vitamin B₁₂ deficiency which have decreased inter-subject variability.

### BRIEF SUMMARY OF THE INVENTION

The present inventors have discovered that vitamin B₁₂, methyl malonic acid (MMA), and/or homocysteine levels can be surprisingly rapidly normalized (for instance, within 15 days of treatment) in a patient having a deficiency of vitamin B₁₂ and/or elevated MMA and/or homocysteine levels when orally administered certain formulations containing sodium *N*-[8-(2-hydroxybenzoyl) amino]caprylic acid (SNAC) and vitamin B₁₂. The inventors have also observed that all patients who have been orally administered the SNAC / vitamin B₁₂ formulation have responded positively to the treatment. The invention is as defined in the claims.

In one aspect of the disclosure, the present invention relates to a method of normalizing vitamin B₁₂ levels (for instance, within 15 days of treatment) in a patient having a vitamin B₁₂ deficiency by administering daily one or more oral dosage forms comprising *N*-[8-(2-hydroxybenzoyl) amino]caprylic acid (NAC) or a pharmaceutically acceptable salt thereof (such as SNAC) and vitamin B₁₂. In one embodiment, the patient suffers from vitamin B₁₂ deficiency due to dietary insufficiency, Intrinsic Factor deficiency, intestinal disturbances, such as malabsorption, gastrectomy, gastric atrophy, ileal resection and chronic inflammatory bowel disease.

In another aspect, the present invention relates to a method of normalizing holotranscobalamin (active vitamin B₁₂) levels (for instance, within 15 days of treatment) in patients with low active B₁₂ levels by administering daily one or more oral dosage forms comprising NAC or a pharmaceutically acceptable salt thereof (such as SNAC) and vitamin B₁₂.

In a further aspect, the present invention relates to a method of reducing MMA levels in a patient having elevated MMA levels by administering daily one or more oral dosage forms comprising NAC or a pharmaceutically acceptable salt thereof (such as SNAC) and vitamin B₁₂.

In yet another aspect, the present invention relates to a method of reducing homocysteine levels in a patient having elevated homocysteine levels by administering daily one or more oral dosage forms comprising NAC or a pharmaceutically acceptable salt thereof (such as SNAC) and vitamin B₁₂.

In yet another aspect, the present invention relates to a method of reducing intersubject variability in the oral treatment of patients suffering from vitamin B₁₂ deficiency and/or elevated MMA and/or homocysteine levels. The method includes, for each patient, administering daily one or more oral dosage forms comprising NAC or a pharmaceutically acceptable salt thereof (such as SNAC) and vitamin B₁₂. In one embodiment, the method achieves a patient response rate similar to or better than that observed for intramuscular administration.

In yet another aspect, the present invention relates to a method of improving the response rate of patients to oral treatment with vitamin B₁₂. The method includes, for each patient, administering daily one or more oral dosage forms comprising NAC\ or a pharmaceutically acceptable salt thereof (such as SNAC) and vitamin B₁₂. In one embodiment, the patients suffer from low vitamin B₁₂ or vitamin B₁₂ deficiency.

In yet another aspect, the present invention relates to a pharmaceutical composition comprising (a) vitamin B₁₂, (b) NAC or a pharmaceutically acceptable salt thereof (such as SNAC), and (c) folic acid. This pharmaceutical composition can be used in any of the aforementioned methods.

In yet another aspect, the present invention relates to a method of reducing or preventing vitamin B₁₂ deficiency induced by a proton pump inhibitor in a patient by orally administering (a) a proton pump inhibitor (such as omeprazole or esomeprazole), (b) vitamin B₁₂, (c) NAC or a pharmaceutically acceptable salt thereof (such as SNAC), and optionally (d) folic acid. All of the components may be in the same or separate dosage forms. In one preferred embodiment, components (b), (c), and optionally (d) are incorporated in a single dosage form, and are co-administered (e.g., simultaneously or within 15 minutes) with the proton pump inhibitor. In another preferred embodiment, all of the components (i.e., components (a), (b), (c), and optionally (d)) are incorporated into a single dosage form. Yet another preferred embodiment is a method of reducing or preventing vitamin B₁₂ deficiency induced by a proton pump inhibitor in a patient being treated with a proton pump inhibitor by (i) discontinuing treatment with the proton pump inhibitor, and (ii) administering (a) a proton pump inhibitor (such as omeprazole or esomeprazole), (b) vitamin B₁₂, (c) NAC or a pharmaceutically acceptable salt thereof (such as SNAC), and optionally (d) folic acid.

In yet another aspect, the present invention relates to a pharmaceutical composition comprising (a) a proton pump inhibitor (such as omeprazole or esomeprazole), (b) vitamin B₁₂, (c) NAC or a pharmaceutically acceptable salt thereof (such as SNAC), and optionally (d) folic acid.

In yet another aspect, the present invention relates to a method of reducing or preventing vitamin B₁₂ deficiency induced by metformin in a patient by orally administering (a) metformin, (b) vitamin B₁₂, (c) NAC or a pharmaceutically acceptable salt thereof (such as SNAC), and optionally (d) folic acid. All of the components may be in the same or separate dosage forms. In one preferred embodiment, components (b), (c), and optionally (d) are incorporated in a single dosage form, and are co-administered (e.g., simultaneously or within 15 minutes) with the metformin. In another preferred embodiment, all of the components (i.e., components (a), (b), (c), and optionally (d)) are incorporated into a single dosage form. Yet another preferred embodiment is a method of reducing or preventing vitamin B₁₂ deficiency induced by metformin in a patient being treated with metformin by (i) discontinuing treatment with metformin, and (ii) administering (a) metformin, (b) vitamin B₁₂, (c) NAC or a pharmaceutically acceptable salt thereof (such as SNAC), and optionally (d) folic acid.

In yet another aspect, the present invention relates to a pharmaceutical composition comprising (a) metformin, (b) vitamin B₁₂, (c) NAC or a pharmaceutically acceptable salt thereof (such as SNAC), and optionally (d) folic acid.

### BRIEF DESCRIPTION OF THE DRAWINGS

Figure 1 shows the mean (SD) percent change from baseline (PCFB) in cobalamin serum concentrations by study day for the subjects described in Example 7.
Figure 2 shows the mean (SD) percent change from baseline (PCFB) in MMA plasma concentrations by study day for the subjects described in Example 7.
Figure 3 shows the mean (SD) percent change from baseline (PCFB) in homocysteine plasma concentrations by study day for the subjects described in Example 7.
Figure 4 is a scatter plot of holo-transcobalamin concentration versus cobalamin concentration by treatment on Day 61 for the subjects described in Example 7.
Figure 5 is a scatter plot of holo-transcobalamin concentration versus cobalamin concentration by treatment on Day 91 for the subjects described in Example 7.
Figure 6 is a bar graph of mean (SD) concentrations for cobalamin by study day for the subjects described in Example 7.

### DETAILED DESCRIPTION

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviations, per practice in the art. Alternatively, "about" with respect to the formulations can mean plus or minus a range of up to 20%, preferably up to 10%, more preferably up to 5%.

As used herein, the term "SNAC" refers to sodium *N*-[8-(2-hydroxybenzoyl) amino]caprylate. SNAC is also known as sodium-N-salicyloyl-8-aminocaprylate, monosodium 8-(N-salicyloylamino) octanoate, N-(salicyloyl)-8-aminooctanoic acid monosodium salt, monosodium N-{8-(2 phenoxybenzoyl)amino}octanoate, E414 monosodium salt, sodium 8-[(2-hydroxybenzoyl)amino]octanoate and salcaprozate. SNAC has the structure:

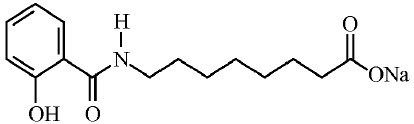

In additional embodiments of any of the methods described herein, NAC or other pharmaceutically salt of SNAC can be used in lieu of SNAC. For example, a disodium salt of NAC may be used. Additionally, any solid state form of SNAC may be used. Suitable solid state form of SNAC are described in U.S. Patent Publication No. 2009/0143330, which is hereby referenced.

In additional embodiments of any of the methods described herein, delivery agents other than SNAC (and its free acid or other pharmaceutically acceptable salts thereof) may be used in combination with vitamin B₁₂. Such delivery agents may either be combined with or used in lieu of NAC or its pharmaceutically acceptable salts. Examples of such delivery agents include, but are not limited to, N-(10-[2-hydroxybenzoyl]amino)decanoic acid (the free acid of SNAD), N-(8-[2-hydroxy-5-chlorobenzoyl]-amino)octanoic acid (5-CNAC), 4-[(2-hydroxy-4-chloro-benzoyl)-amino]butanoic acid (4-CNAB), 8-(2-hydroxyphenoxy)octyldiethanolamine (HPOD), 8-(N-2-hydroxy-4-methoxybenzoyl)-aminocaprylic acid (4-MOAC), and pharmaceutically salts thereof (e.g., monosodium and disodium salts thereof). The term "SNAD" refers to the monosodium salt of N-(10-[2-hydroxybenzoyl-]amino)decanoic acid. Other suitable delivery agents are described, for example, in International Publication Nos. WO 96/30036, WO 98/34632, and WO 2007/121318 and U.S. Patent Nos. 5,650,386, 5,773,647, and 5,866,536, all of which are hereby referenced.

The term "vitamin B₁₂" refers to any member of a group of cobalt-containing compounds known as cobalamins which include, but is not limited to, cyanocobalamin, hydroxocobalamin, methylcobalamin, and 5-deoxyadenosylcobalamin. In one embodiment, the vitamin B₁₂ is cyanocobalamin.

The patient has a serum vitamin B₁₂ level below 350, pg/mL.

In certain embodiments of any of the methods described herein, the patient has a plasma holotranscobalamin (active vitamin B₁₂) level below about 100 pmol/L. For example, the patient can have a plasma active vitamin B₁₂ level below about 50 or about 25 pmol/L.

In certain embodiments of any of the methods described herein, the patient has a serum MMA level of at least about 150 nmol/L. For example, the patient can have a serum MMA level of at least about 175 nmol/L, at least about 200 nmol/L or at least about 225 nmol/L.

In certain embodiments of any of the methods described herein, the patient has a plasma homocysteine level of at least about 13 □mol/L. For example, the patient can have a plasma homocysteine level of at least about 14 □mol/L.

In another aspect, the present invention relates to a method of reducing intersubject variability in the oral treatment of patients suffering from low vitamin B₁₂ or vitamin B₁₂ deficiency, the method comprising, for each patient, daily administering one or more oral dosage forms comprising *N*-[8-(2-hydroxybenzoyl) amino]caprylic acid (NAC) or a pharmaceutically acceptable salt thereof (such as SNAC) and vitamin B₁₂.

In the methods of the present invention, the oral dosage form of NAC (or a pharmaceutically acceptable salt thereof) and vitamin B₁₂ is preferably administered daily for at least 15, 30, 60, or 90 days.

In yet another aspect, the present invention relates to a method of improving the response rate of patients to oral treatment with vitamin B₁₂ by daily administering to each patient one or more oral dosage forms comprising *N*-[8-(2-hydroxybenzoyl) amino]caprylic acid (NAC) or a pharmaceutically acceptable salt thereof (such as SNAC) and vitamin B₁₂. In one embodiment, the patients suffer from low vitamin B₁₂ or vitamin B₁₂ deficiency.

In one embodiment, any of the methods described herein achieve a patient response rate similar to or better than that observed for intramuscular administration.

In a further aspect, the present invention relates to pharmaceutical compositions comprising (a) vitamin B₁₂, (b) *N*-[8-(2-hydroxybenzoyl) amino]caprylic acid (NAC) or a pharmaceutically acceptable salt thereof (such as SNAC), and (c) optionally, one or more additional biologically active agents useful in any of the methods described herein. Examples of suitable additional active agents include, but are not limited to, folic acid. In one embodiment, the weight ratio of NAC (or a pharmaceutically acceptable salt thereof, such as SNAC) to vitamin B₁₂ in the pharmaceutical composition is from about 25:1 to about 500:1.

In one embodiment, the present invention relates to an oral pharmaceutical composition comprising (a) vitamin B₁₂, (b) *N*-[8-(2-hydroxybenzoyl) amino]caprylic acid (NAC) or a pharmaceutically acceptable salt thereof (such as SNAC) and (c) folic acid. The weight ratio and amount of vitamin B₁₂ and SNAC (or other form of NAC) can be as described herein.

In additional embodiments, the amount of folic acid in the composition ranges from about 0.05 □g to about 1000 □g. For example, from about 1 □g to about 800 □g, from about 10 □g to about 800 □g, from about 100 □g to about 800 □g, from about 250 to about 800 □g, from about 250 to about 500 □g or from about 400 □g to about 800 □g. For example, the amount of folic acid in the composition may be about 200 □g, about 250 □g, about 300 □g, about 400 □g, about 500 □g, about 600 □g, about 700 □g or about 800 □g.

In additional embodiments of any of the methods described herein, the patient is one who has failed to respond to existing oral vitamin B₁₂ treatment (or, for instance, oral treatment with a vitamin B₁₂ formulation which does not include SNAC).

In further embodiments of any of the methods described herein, the present invention relates to the administration of a tablet dosage form.

In other embodiments, the dosage form (e.g., a tablet) optionally contains excipients, emulsifiers, stabilizers, sweeteners, flavoring agents, diluents binders coloring agents and/or solubilizing agents, lubricants (such as magnesium stearate), or any combination thereof. Suitable excipients, emulsifiers, stabilizers, sweeteners, flavoring agents, diluents, coloring agents, and solubilizing agents include those described in the Handbook of Pharmaceutical Excipients (fourth edition) by Raymond C. Rowe, Paul J. Sheskey and Paul J. Weller.

Without intending to be bound by any particular theory of operation, it is believed that gastrointestinal absorption of vitamin B₁₂ depends on the presence of sufficient intrinsic factor protein, secreted from gastric parietal cells. The average diet supplies about 10 mcg/day of vitamin B₁₂ in a protein-bound form that is available for absorption after normal digestion. Vitamin B₁₂ is bound to intrinsic factor during transit through the stomach; separation occurs in the terminal ileum, and vitamin B₁₂ enters the mucosal cell for absorption via a receptor mediated process. It is then transported by the transcobalamin binding proteins. A small amount (approximately 1% of the total amount ingested) is absorbed by simple diffusion, but this mechanism is adequate only with very large doses. It is also believed that SNAC allows vitamin B₁₂ to bypass its usual receptor mediated process.

The term "treatment" or "treating" means any treatment of a disease or disorder in a mammal, including: preventing or protecting against the disease or disorder, that is, causing the clinical symptoms not to develop; inhibiting the disease or disorder, that is, arresting or suppressing the development of clinical symptoms; and/or relieving the disease or disorder, that is, causing the regression of clinical symptoms.

The patient may suffer from vitamin B₁₂ deficiency due to malabsorption which may be associated with the following conditions: (1) addisonian (pernicious) anemia, (2) gastrointestinal pathology, dysfunction, or surgery, including gluten enteropathy or sprue, small bowel bacteria overgrowth, (3) total or partial gastrectomy, (4) fish tapeworm infestation, (5) malignancy of pancreas or bowel, or (6) folic acid deficiency. For instance, the patient may have (1) a dietary deficiency of vitamin B₁₂, (2) pernicious anemia, (3) malabsorption of vitamin B₁₂ from functional disturbances of intrinsic factor mediated absorption, gastrectomy, inflammatory bowel disease, age related disturbances of dietary vitamin B12 absorption, bacterial overgrowth of the intestine or the chronic use of proton pump inhibitors or metformin, or (4) conditions affecting the small intestine, such as tropical sprue, Crohn's disease, chronic alcoholism, abdominal or intestinal surgery that impacts Intrinsic Factor production. Manifestations of B₁₂ deficiency include pernicious anemia and neurologic degeneration. Additionally, poor B₁₂ status has been linked to health-related conditions such as poor cognition, Alzheimer's disease, depression, and poor bone health. The patient may also suffer from vitamin B₁₂ deficiency due to the chronic use of certain drugs, such as proton pump inhibitors or metformin which lead to vitamin B₁₂ deficiency.

The methods of the present invention are also useful for providing patients which require in excess of normal amounts of vitamin B₁₂, such as due to pregnancy, thyrotoxicosis, hemolytic anemia, hemorrhage, malignancy, hepatic and renal disease.

The term "intrinsic factor protein" refers to a glycoprotein produced by the parietal cells of the stomach. This glycoprotein facilitates the absorption of vitamin B₁₂ later on in the terminal ileum.

Normal serum vitamin B₁₂ concentrations typically range, for example, from about 200-900 pg/mL, with a mean normal plasma concentration of about 450 pg/mL. See, e.g., AHFS Drug Information: McEvoy, G.K. ed. American Society of Health-System Pharmacists, 2007). Serum vitamin B₁₂ concentrations less than about 200 pg/mL indicate vitamin B₁₂ deficiency, and serum B₁₂ concentrations ranging from about 200 to about 350 pg/mL, when combined with elevated MMA and total homocysteine levels, may be considered signs of a depleting tissue store. See, e.g., Carmel R, et al., Hematology Am. Soc. Hematol. Educ. Program, 62-81, 2003. Such subjects may be treated by the methods described herein. When serum B₁₂ levels are below about 350-400 pg/mL, subjects may be considered borderline B₁₂ deficient. See, e.g., Cravens D, et al., Journal of Family Practice, 56(1) 62-63, 2007; Ramsay, ID et al., Clin. Exp. Dermatol., 15(4), 277-81, 1990. Such subjects may be treated by the methods described herein.

Suitable proton pump inhibitors include, but are not limited to, omeprazole, hydroxyomeprazole, esomeprazole, tenatoprazole, lansoprazole, pantoprazole, rabeprazole, dontoprazole, habeprazole, perprazole, ransoprazole, pariprazole, leminoprazole, timoprazole, disulprazole, and pharmaceutically acceptable salts thereof. In one embodiment, the oral dosage form contains from about 5 to about 60 mg of any of these proton pump inhibitors (such as omeprazole, esomeprazole, or pantoprazole). In one preferred embodiment, the oral dosage form contains 10, 20, or 40 mg of the proton pump inhibitor. In another embodiment, the daily dosage of any of these proton pump inhibitors (such as omeprazole, esomeprazole, or pantoprazole) ranges from about 5 to about 60 mg daily. In one preferred embodiment, the daily dosage is 10, 20, or 40 mg of the proton pump inhibitor.

Oral dosage forms containing a proton pump inhibitor or metformin may have the proton pump inhibitor or metformin in a layer separate from the NAC (or salt thereof) and vitamin B12. This permits the proton pump inhibitor or metformin layer to provide a different release provide (for example, immediate or extended release) than the vitamin B₁₂ / NAC layer.

A subject or patient in whom administration of the oral pharmaceutical composition is an effective therapeutic regimen for a disease or disorder is preferably a human, but can be any animal, including a laboratory animal in the context of a trial or screening or activity experiment. Thus, as can be readily appreciated by one of ordinary skill in the art, the methods and compositions of the present invention are particularly suited to administration to any animal, particularly a mammal (e.g., a human), and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., i.e., for veterinary medical use.

The following examples are given as specific illustrations of the invention. It should be understood, however, that the invention is not limited to the specific details set forth in the examples. All parts and percentages in the examples, as well as in the remainder of the specification, are by weight unless otherwise specified.

Further, any range of numbers recited in the specification or paragraphs hereinafter describing or claiming various aspects of the invention, such as that representing a particular set of properties, units of measure, conditions, physical states or percentages, is intended to literally incorporate expressly herein by reference or otherwise, any number falling within such range, including any subset of numbers or ranges subsumed within any range so recited.

### Example 1. Preparation of N-[8-(2-hydroxybenzoyl) amino]caprylic acid and SNAC

The preparation method for *N*-[8-(2-hydroxybenzoyl) amino]caprylic acid and SNAC involves the following steps: The starting material is salicylamide, which is converted to form carsalam (1,3-benzoxazine-2,4-dione). The second step involves the alkylation of carsalam. The penultimate step is a hydrolysis to cleave the ethyl protection group at the end of the alkyl chain and open the heterocyclic ring forming the free acid of SNAC. In the final step, the sodium salt of the SNAC free acid is formed by reaction with a 1% excess stoichiometric amount of sodium hydroxide base. Upon cooling the precipitated product is isolated by centrifugation and vacuum dried prior to packaging. The in-process controls for the synthetic scheme are given in Table 1.

**Table 1. In-process controls for SNAC Manufacturing Process.**

| **Step** | **Reaction** | **Desired Product** | **Specification** | **In-Process Control** |
|---|---|---|---|---|
| 1 | Carsalam | Carsalam | <10% salicylamide | HPLC |
| 2 | Alkylation | Alkylated carsalam | <8% carsalam | HPLC |
| 3 | Hydrolysis | SNAC free acid | <0.5% | LOD |
| 4 | Sodium Salt | SNAC sodium salt | 95-105% | HPLC |

### Example 2. Preparation of Vitamin B₁₂ Tablets (comparative).

The tablet die and punches are checked to ensure that they are clean and that their surfaces are dusted with magnesium stearate powder. Vitamin B₁₂, SNAC, carrier, excipient, emulsifier, stabilizer, sweetener, flavoring agent, diluent, coloring agent, solubilizing agent are screened through a #35 sieve and transferred into a sealed container. 50 mg of vitamin B₁₂ is weighed and mixed thoroughly with 11 grams of a carrier, excipient, emulsifier, stabilizer, sweetener, flavoring agent, diluent, coloring agent and/or solubilizing agent. 100 vitamin B₁₂ tablets are made, with each tablet containing 0.5 mg of vitamin B₁₂ and 110 mg of a carrier, excipient, emulsifier, stabilizer, sweetener, flavoring agent, diluent, coloring agent and/or solubilizing agent. These tablets are used as a control.

### Example 3. Preparation of Vitamin B₁₂ and SNAC-Tablets (comparative).

50 mg of vitamin B₁₂ and 1 gram of SNAC are weighed and thoroughly mixed with 10 grams of a carrier, excipient, emulsifier, stabilizer, sweetener, flavoring agent, diluent, coloring agent and/or solubilizing agent. 100 vitamin B₁₂ tablets are made, with each tablet containing 0.5 mg of vitamin B₁₂, 10 mg of SNAC and 100 mg of a carrier, excipient, emulsifier, stabilizer, sweetener, flavoring agent, diluent, coloring agent and/or solubilizing agent. The process is repeated to make tablet batches containing 1.0 mg, 0.8 mg, 0.6 mg, 0.4 mg and 0.2 of vitamin B₁₂, respectively.

These tablets have the specifications for release of the SNAC component as shown in Table 2:

**Table 2**

| **Tests** | **Specification** | **Analytical Method** |
|---|---|---|
| Appearance | White to light-tan powder with pink hue | AM001 |
| Identification Test for sodium FTIR | Confirms presence of sodium Conforms to reference standard | USP <191 > |
| | | USP <197K> |
| Melting Range/Temperature | 193 - 203 °C with a range not to exceed 5°C | USP <741> |
| Water Content | NMT 3.0 % | USP <921> Method I |
| Heavy Metals | < 20 ppm | USP <231> Method II |
| Sodium Content | 6.9 to 8.4 % | AM017 |
| Residual Solvents | | |
| Ethanol | Less than 4000 ppm | AM008 |
| Heptane | Less than 500 ppm | AM008 |
| Assay as SNAC sodium salt (as is) | 90.0 -110.0 % w/w | AM016 |

### Example 4. Preparation of Tablets for Testing on Rats

Tablets with four types of different ingredients were made as follows:

(1) 8.8 mg of vitamin B₁₂, 35 mg of SNAC were weighed, thoroughly mixed and made into a tablet for dosing in rats; (2) 8.8 mg of vitamin B₁₂, 35 mg of SNAC and 5 mg of capmul PG-8 (propylene glycol monocaprylate) were weighed, thoroughly mixed and made into a tablet; (3) 8.8 mg of vitamin B₁₂, 35 mg of SNAC and 0.9 mg of povidone were weighed, thoroughly mixed and made into a tablet. Each of the four processes was repeated to produce more tablets.

### Example 5. Dosing Sprague-Dawley Rats

Male Sprague-Dawley rats (325 - 350g) were dosed with vitamin B₁₂ intravenously (0.5 mg/kg) alone, or orally with the tablets made in Example 4 at a dose of 50 mg/kg, vitamin B₁₂ alone or in combination with SNAC at 200 mg/kg. Blood samples were collected at 0, 3, 10, 20, 30, 60, 120, 240 and 360 minutes post dosing. Plasma samples were analyzed for vitamin B₁₂ by RIA. The model independent pharmacokinetic (PK) metrics obtained following administration of the vitamin B₁₂/SNAC combination were compared to those obtained following administration of vitamin B₁₂ alone. The results are shown in Table 3.

**Table 3. Comparative Testing Results for Vitamin B₁₂ Absorption**

| Group (N=5) | *Cmax (ug*/*mL)* | | *Tmax (min)* | | *AUC (ug^{∗}min*/*mL)* | | Mean Bioavailability |
|---|---|---|---|---|---|---|---|
| | Mea n | S.D | Mea n | S.D | Mean | S.D | % |
| 0.5mg/kg Vitamin B₁₂ (IV) | 2.15 | 0.64 | 4.4 | 3.13 | 65.84 | 11 | |
| 50mg/kg Vitamin B₁₂ alone (PO) | 0.14 | 0.07 | 52 | 17.9 | 28.72 | 13 | 0.42 |
| 50mg/kg Vitamin B₁₂ + 200mg/kg SNAC (PO) | 7.99 | 2.41 | 24 | 5.48 | 522.37 | 179 | 7.93 |

### Example 6. Preparation of Tablets for Testing on Human Subjects (comparative).

Tablets were made from cyanocobalamin, SNAC, Kollidon 90F, Anhydrous Emcompress USP/EP and Magnesium Stearate, NF/BP/EP/JP. Each tablet contains the following ingredients, as shown in Table 4:

**Table 4**

| **Ingredient** | **mg/tablet** |
|---|---|
| Cyanocobalamin, USP (Intragranular) | 5.00 |
| SNAC (Intragranular) | 100.00 |
| Kollidon 90F, NF/EP/JP (Povidone K90; Intragranular) | 2.00 |
| Anhydrous Emcompress USP/EP (Diabasic Calcium Phosphate, Anhydrous; Intragranular) | 70.00 |
| Anhydrous Emcompress USP/EP (Diabasic Calcium Phosphate, Anhydrous; Extragranular) | 21.00 |
| Magnesium Stearate, NF/BP/EP/JP (extragranular) | 2.00 |
| Total Weight | 200.0 |

### Example 7: Open Label Randomized Study

In this study, the efficacy and safety of the oral vitamin B₁₂ formulation (1 mg) of the present invention and intramuscular (IM) cyanocobalamin (1 mg) were evaluated in subjects with mild vitamin B₁₂ deficiency.

### Study Design:

This was an open-label, randomized 60-day study, with a 30-day extension, conducted in B₁₂ deficient subjects. Subjects were assigned to a treatment according to a randomization schedule prepared by a statistician at the start of the study. The randomization scheme was stratified such that a balanced number of males and females were assigned to each treatment group. Subjects were randomized to receive either a 1000 µg oral tablet, administered once daily for 90 days or 1000 µg cyanocobalamin, administered IM on Days 1, 3, 7, 10, 14, 21, 30, 60 and 90.

A single tablet was self-administered in the fasted state and at least one hour prior to the morning meal with 50 mL water. IM study drug administration was performed by study personnel, in the research clinic, in the morning, in the fasted state and at least 1 hour prior to the morning meal on study Days 1, 3, 7, 10, 14, 21, 30, 60 and 90. For subjects assigned to receive tablets, treatment compliance was based on subject diary entries. Blood samples for pharmacodynamic assessments (efficacy variables) were collected from all subjects at baseline prior to dosing (Day 1), and on Days 15, 31, 61 and 91. Sample collection was carried out approximately 24 hours after the last dose administration, prior to that day's dose administration, and in the fasting state.

### Materials:

Sterile cyanocobalamin for injection, USP 1000 µg/mL was used as individual 1mL vials.

The composition of the tablets administered to the subjects is shown in Table 5

**Table 5**

| **Ingredient** | **mg/tablet** |
|---|---|
| Cyanocobalamin, USP | 1.00 |
| SNAC | 100.00 |
| Povidone | 2.00 |
| Dibasic calcium phosphate | 95.00 |
| Magnesium stearate | 2.00 |
| Total weight (mg) | 200.00 |

### Subjects:

A total of 50 adult male and female subjects were randomized to receive study drug. Twenty-two of the 24 subjects (91.7%) randomized to receive tablets completed the study as planned. Twenty-six subjects were randomized to receive IM B₁₂ and 26 (100%) of subjects completed the study as planned. These subjects suffered from mild vitamin B12 deficiency.

Eligible subjects, as per inclusion criteria, were male or female whose clinical laboratory data showed vitamin B₁₂ deficiency defined as serum cobalamin below 350 pg/mL. In order to enter the study, subjects were required to be age 60 or older; or age 18 or older with gastrointestinal abnormalities including but not limited to gastrointestinal surgery (e.g. gastrectomy, gastric bypass), ileal resection, gastric atrophy, Celiac disease, Crohn's disease, or prolonged use (>3 months) of proton pump inhibitor drugs, or on a restricted diet (such as vegetarian or vegan). Additional inclusion criteria were general good health, as indicated by lack of significant findings in medical history, physical examination, clinical laboratory tests, vital signs, ECG and normal kidney function as determined by estimated creatinine clearance computed with the Cockcroft and Gault formula, since impaired renal function may elevate homocysteine (HC), methylmalonic acid (MMA) and holotranscobalamin values (Herrmann et al., Clin Chem Lab Med 2003; 41: 1478-88). Exclusion criteria included current treatment from a health care provider to treat vitamin B₁₂ deficiency and/or symptoms; daily use of neutralizing antacids (e.g. Maalox®); inability to ingest oral medication; any clinically significant laboratory value at screening; hypersensitivity or allergic reaction to vitamin B₁₂; participation in a clinical research study involving a new chemical entity within 30 days of the first study dose; and folate levels below the reference range provided by the clinical laboratory.

Confounding factors in the diagnosis and treatment of vitamin B₁₂ deficiency include folate deficiency, renal insufficiency and vitamin B₆ deficiency. Folate deficiency can cause many of the same symptoms as B₁₂ deficiency such as elevated total HC. Renal insufficiency may elevate MMA levels in the blood, and a vitamin B₆ deficiency or hypothyroidism may lead to elevated total HC. These clinical parameters of B₁₂ deficiency, when attributed to other causes, would not respond to B₁₂ treatment. Subjects with the above deficiencies were excluded from the study.

Subjects recorded concomitant medications (including vitamins, herbal supplements and antacids) and adverse events in diaries distributed on study Day 1. Subjects randomized to receive oral treatment also recorded dosing information (date and time) and time of meals consumed before and after each dose.

### Treatments:

The 1000 µg oral tablet was taken in the fasted state as a single tablet with 50 mL water. Each dose was self-administered daily, for 90 days, after an overnight fast and 1 hour before the morning meal. No liquid was consumed for at least 1 hour before and 1 hour after dosing.

1000 µg cyanocobalamin was administered IM as 1 mL from a vial containing 1000 µg/mL drug. Study drug administration was performed by study personnel, in the research clinic, in the morning, in the fasted state and at least 1 hour prior to the morning meal on study Days 1, 3, 7, 10, 14, 21, 30, 60 and 90.

### Blood sample processing:

Serum cobalamin (B₁₂) levels were determined by a validated microparticle enzyme immunoassay (MEIA) detection method with a calibration range of 100-2000 pg/mL. Sample dilution procedures were validated up to 4-fold dilution. Serum MMA levels were determined by a validated LC/MS/MS method with a calibration range of 5-200 ng/mL. Plasma HC levels were determined by a validated fluorescence polarization immunoassay (FIPA) detection method with a calibration range of 2.5-50 µmol/L; and plasma holotranscobalamin (active B₁₂) levels were determined using a validated MEIA method, with a calibration range of 8-128 pmol/L. Sample dilution procedures were validated up to 32-fold dilution.

### Safety Assessments:

Safety assessments consisted of monitoring adverse events (AEs), hematology, chemistry and urinalysis laboratory test results, concomitant medications, vital sign assessments, ECG and physical examination findings.

A resting 12-lead ECG was recorded at screening and at the End-of-Study visit prior to discharge from the study. The ECG parameters recorded included ventricular rate (bpm), PR interval (msec), QRS duration (msec), QT interval (msec), QTc interval (msec, Bazett's correction). A physical examination was performed at screening and at the End-of-Study visit prior to discharge from the study.

### Efficacy Assessments:

The primary efficacy variable was serum cobalamin levels on Day 61. The primary efficacy outcome compared the proportion of subjects in each treatment group whose cobalamin levels were normalized (≥350 pg/mL) on Day 61. The secondary efficacy variables were serum cobalamin levels on Day 91, serum MMA levels on Day 61 and Day 91 and, plasma total HC levels on Day 61 and Day 91.

The secondary efficacy outcomes were: comparison of the proportion of subjects in each treatment group whose cobalamin levels were normalized (≥350 pg/mL) on Day 91; comparison of the mean percent change from baseline in serum cobalamin levels among subjects in each treatment group on Day 61 and Day 91; comparison of the mean percent change from baseline in serum MMA levels among subjects in each treatment group on Day 61 and Day 91; comparison of the mean percent change from baseline in total plasma HC among subjects in each treatment group on Day 61 and Day 91; comparison of the mean first time to normalization of serum cobalamin (≥350 pg/mL) among subjects in each treatment group on Day 61 and Day 91. Exploratory efficacy variables and outcomes were: plasma holotranscobalamin levels on Day 61 and Day 91, measured by: comparison of the proportion of subjects in each treatment group whose holotranscobalamin levels were normalized (≥40 pmol/L) on Day 61 and Day 91; the relationship between cobalamin and holotranscobalamin levels on Day 61 and Day 91.

### Statistical Analysis:

Programming and statistical analyses were conducted using SAS (Version 9.1, SAS Institute, Cary, North Carolina, USA).

Three analysis populations were used. The Intent-to-treat (ITT) population included all subjects randomized into the trial, regardless of whether they received study product. Analyses using the ITT population assigned subjects to the group to which they were randomized. The per-protocol population included all randomized subjects who received at least 90% of their assigned study treatment, who had non-missing baseline and Day 61 serum cobalamin assessments, and who met all inclusion criteria and no exclusion criterion. The principal analyses were conducted on the ITT population.

The safety population included all randomized subjects who received at least one administration of study product and who had at least one subsequent safety assessment. Subjects are included in this group based on the actual treatment received. All safety analyses were performed on the safety population.

The analysis of the primary efficacy outcome compared the proportion of subjects in each treatment group whose cobalamin levels were normalized (i.e., cobalamin ≥ 350 pg/mL) on Day 61 Visit. The number and frequency of normalized subjects was calculated. The 90% confidence interval of the difference in the proportions (tablet - IM B12) was calculated and presented using exact procedures. A conclusion of non-inferiority was made if the lower bound of the two-sided 90% exact confidence interval for the difference between the two proportions (tablet - IM B12) exceeded -0.15.

The analyses for all secondary outcomes used only available data (i.e., no imputation for missing data occurring after first dose of study medication). For all tests of secondary hypotheses, a p-value < 0.05 indicated a statistically significant result. No adjustments were made for multiplicity. For subjects with missing baseline data, the latest screening value was used as baseline for the calculation of change from baseline and percent change from baseline.

Change from baseline (CFB) is defined as (X-B). Percent change from baseline (PCFB) is defined as PCFB=100(X-B)/B, where B is the baseline (pre-dose Day 1) measurement and X is the measurement at Day 61 or Day 91, as required.

The proportion of subjects who achieved normalization of cobalamin levels (i.e., cobalamin ≥ 350 pg/mL) at Day 91 was to be analyzed using the same methodology as given above for the primary analysis (i.e., Day 61). The PCFB for cobalamin levels at Days 61 and 91 was analyzed using ANCOVA with treatment group and baseline cobalamin measurement as fixed effects. For each outcome and each time point the mean difference between the groups was be tested. The PCFB for MMA and HC levels at Days 61 and 91 was analyzed using ANCOVA with treatment group and baseline MMA or HC measurement as fixed effects. For each outcome and each time point the mean difference between the groups was tested.

Time to first normalization of cobalamin (≥ 350 pg/mL) from Day 1 to Day 61 and from Day 1 to Day 91 was analyzed using a log-rank test. The Kaplan-Meier curves, hazard ratio and its 95% confidence interval were calculated. Time zero was taken as Study Day 1.

The proportion of subjects who achieved normalization of holotranscobalamin levels (≥40 pmol/L) at Day 61 and Day 91 were analyzed using the same methodology as given for the primary analysis. The relationship between cobalamin and holotranscobalamin levels at Day 61 and Day 91, and the PCFB to Day 61 and Day 91 were explored. The relationship was analyzed graphically.

### Results:

### Patient Demographic and Baseline

A total of 50 healthy subjects [11 (22.0%) male, 39 (78.0% female] were randomized to receive study drug. Mean (SD) age was 53.2 (15.33) years and race was 40 (80.0%) Caucasian and 10 (20.0%) Black. Mean (SD) serum cobalamin levels at screening were similar among subjects randomized to receive oral vitamin B₁₂ [285.5 (54.27) pg/mL] compared to subjects randomized to receive IM B₁₂ [262.0 (54.61) pg/mL].

### Efficacy

The proportion of subjects in each treatment group whose cobalamin levels were normalized (≥350 pg/mL) on Day 61 (primary efficacy outcome) and Day 91 (secondary efficacy outcome) are presented in Table 6 (ITT population). Results were comparable for both populations.

**Table 6 - Cobalamin Responders**

| Study day | Statistics | Treatment group | |
|---|---|---|---|
| | | Oral B₁₂ (N=24) | IM B₁₂ (N=26) |
| Day 15 | N | 23 | 26 |
| | Responder (%)¹ | 23 (100.0) | 26 (100.0) |
| | Non-responder (%) | 0 (0.0) | 0 (0.0) |
| Day 61 | N | 23 | 26 |
| | Responder (%)¹ | 23 (100.0) | 26 (100.0) |
| | Non-responder (%) | 0 (0.0) | 0 (0.0) |
| Day 91 | N | 22 | 26 |
| | Responder (%)¹ | 22 (100.0) | 26 (100.0) |
| | Non-responder (%) | 0 (0.0) | 0 (0.0) |

| | | | |
|---|---|---|---|
| ¹- A responder is defined as a subject having a cobalamin level >= 350 pg/mL. There are 13 subjects in the oral B₁₂ group and 14 subjects in the IM B₁₂ group with >=350 pg/mL cobalamin level at baseline. | | | |

All subjects in each treatment group achieved normalization of serum cobalamin levels (i.e., cobalamin levels ≥ 350 pg/mL) by Day 15.

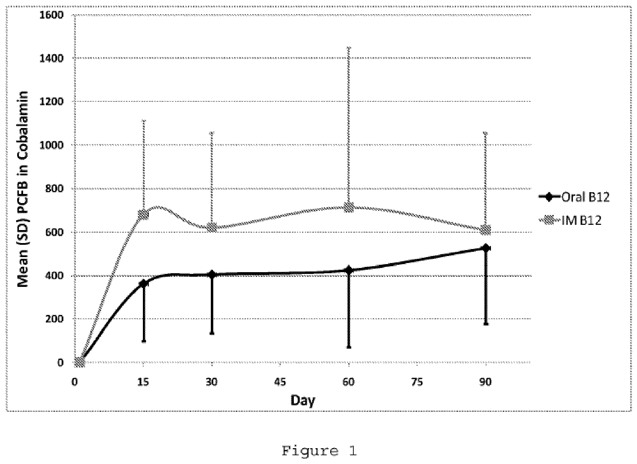

There was no statistical difference between treatment groups for mean percent change from baseline (PCFB) in serum cobalamin levels on Day 61 (p=0.1326) and Day 91 (p=0.4273). On Day 61, the mean (SD) percent change in cobalamin levels was 452.5 (353.19) and 714.6 (732.11) for subjects in the oral B₁₂ and IM B₁₂ treatment groups, respectively. On Day 91, the mean (SD) percent change in cobalamin levels was 526.9 (350.62) and 608.9 (446.68) for subjects in the oral B₁₂ and IM B₁₂ treatment groups, respectively. The results were identical for the ITT population and for the per protocol population. Figure 1 shows the mean PCFB in cobalamin levels by study day for the ITT population. As can be seen from Figure 1, the oral B₁₂ group surprisingly showed normalized cobalamin serum levels at the first time point (Day 15). These subjects maintained normalized average serum vitamin B₁₂ levels throughout the duration of the study, up to and including Day 91.

Subjects in the oral B₁₂ treatment group surprisingly showed normalized active vitamin B₁₂ levels at the first time point (Day 15). These subjects maintained normalized active vitamin B₁₂ levels throughout the duration of the study, up to and including Day 91.

There was no statistical difference between treatment groups for mean PCFB in serum MMA levels on Day 61 (p=0.6179). On Day 61, the mean (SD) percent change in MMA levels was -30.55 (26.655) and -28.17 (24.754) for subjects in the oral B₁₂ and IM B₁₂ treatment groups, respectively.

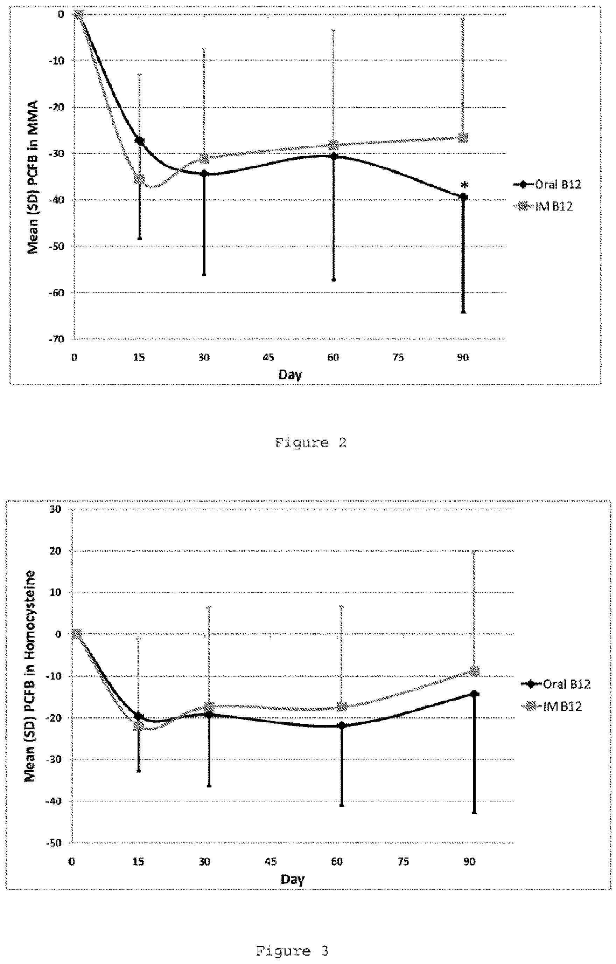

On Day 91, the oral treatment group exhibited a mean PCFB in serum MMA levels that was superior to the IM B₁₂ group (p=0.0334). On Day 91, the mean (SD) percent change in MMA levels was -39.29 (24.939) and -26.58 (25.643) for subjects in the oral B₁₂ and IM B₁₂ treatment groups, respectively. Figure 2 shows the mean PCFB in MMA levels by study day for the ITT population. Subjects in the oral B₁₂ treatment group surprisingly showed normalized methyl malanonic acid levels at the first time point (Day 15). These subjects maintained normalized MMA levels throughout the duration of the study, up to and including Day 91.

There was no statistical difference between treatment groups for mean PCFB in plasma HC levels on Day 61 (p=0.6368) and Day 91 (p=0.5140). On Day 61, the mean (SD) percent change in HC levels was -21.94 (19.034) and -17.49 (24.099) for subjects in the oral B₁₂ and IM B₁₂ treatment groups, respectively. On Day 91, the mean (SD) percent change in HC levels was - 14.36 (28.375) and -8.805 (28.5406) for subjects in the oral B₁₂ and IM B₁₂ treatment groups, respectively. Figure 3 shows the mean PCFB in HC levels by study day for the ITT population. Subjects in the oral B₁₂ treatment group surprisingly showed normalized homocysteine acid levels at the first time point (Day 15). These subjects maintained normalized homocysteine levels throughout the duration of the study, up to and including Day 91.

The proportion of subjects in each treatment group whose plasma holotranscobalamin levels were normalized (≥ 40 pmol/L) on Day 61 and Day 91 (exploratory outcome) are presented in Table 7 (ITT population).

**Table 7 - Holotranscobalamin Responders**

| Study day | Statistics | Treatment group | |
|---|---|---|---|
| | | Oral B₁₂ (N=24) | IM B₁₂ (N=26) |
| Day 15 | n | 23 | 26 |
| | Responder (%)¹ | 23 (100.0) | 26 (100.0) |
| | Non-responder (%) | 0 (0.0) | 0 (0.0) |
| Day 61 | n | 22 | 26 |
| | Responder (%)¹ | 22 (100.0) | 26 (100.0) |
| | Non-responder (%) | 0 (0.0) | 0 (0.0) |
| Day 91 | N | 22 | 26 |
| | Responder (%)¹ | 22 (100.0) | 26 (100.0) |
| | Non-responder (%) | 0 (0.0) | 0 (0.0) |

| | | | |
|---|---|---|---|
| ¹- A responder is defined as a subject having a Holotranscobalamin level >= 40 pmol/L. There was no sample for one subject as of Day 61 and therefore that subject was not counted in the responder or non-responder categories. | | | |

All (100%) subjects in both treatment groups, and in both populations, had plasma holotranscobalamin levels ≥40 pmol/L on Day 61 and on Day 91.

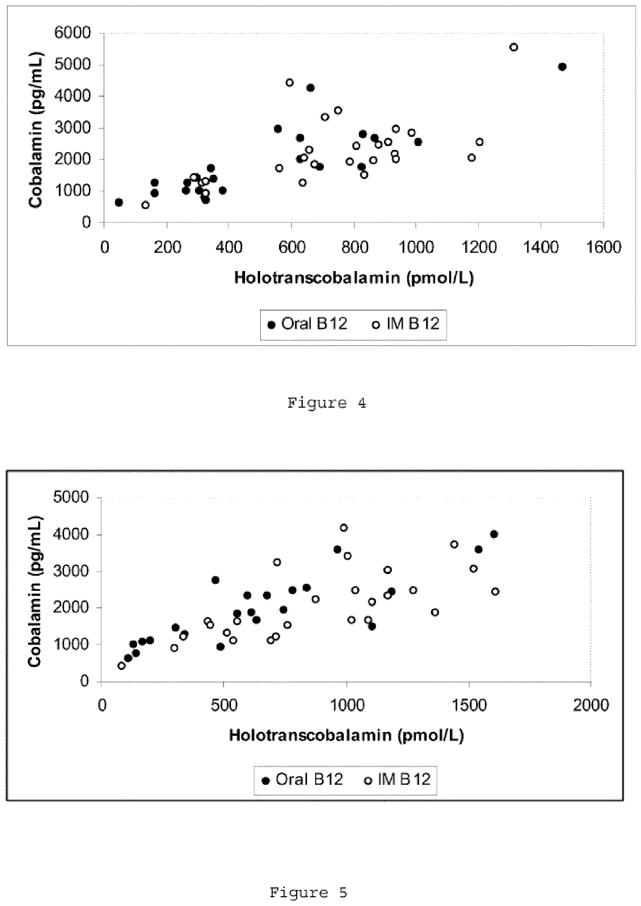

Scatter plots of holotranscobalamin levels in relation to cobalamin levels on Days 61 and 91 for subjects in each treatment group in the ITT population are provided in Figures 4 and 5. In general, it appears that for subjects in each treatment group as cobalamin levels increase, an increase in holotranscobalamin levels is observed on Day 61 and on Day 91.

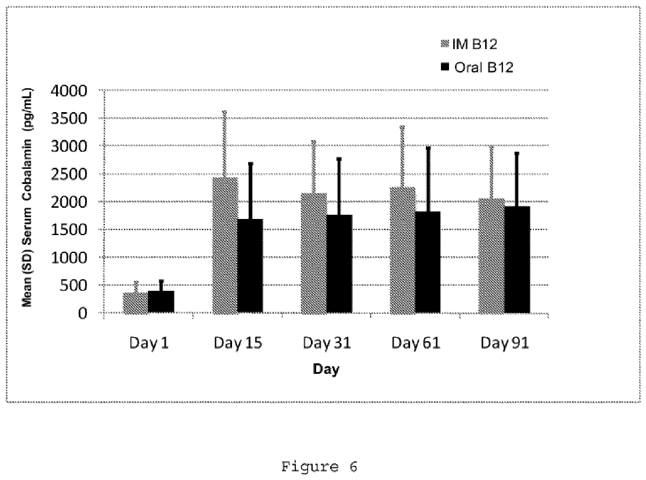

Table 5 and 6 present cobalamin, holotranscobalamin, MMA and HC individual data following IM and oral treatment, respectively. Figure 6 is a bar graph of mean (SD) serum cobalamin concentrations at baseline (Day 1) and Days 15, 31, 61, and 91 following both treatments. All enrolled patients screened by the clinical laboratory had cobalamin values <350 pg/mL. When the validated cobalamin method was used 13 subjects in Eligen® B12 and 14 subjects in the IM group were > or = to 350 pg/mL. Most of these patients were within 10% of 350 pg/mL. All of the subjects showed cobalamin and holotranscobalamin normalization at day 61 and normalization was maintained at day 91. Biomarkers, MMA and HC, were decreased in patients who had elevated levels at screening (subjects 102, 103, 202, 203 and 239 for the IM group and 101, 228, 232 and 233 for the oral group). A decrease was observed in most of the patients whose biomarker concentrations were in the normal range.

**Table 8 - IM Treatment Group**

| Parameter | Day | Mean | Standard Deviation (SD) | Percent Co-variance (%CV) |
|---|---|---|---|---|
| Total B₁₂ (pg/mL) | 1 | 369 | 180 | 49 |
| | 15 | 2434 | 1181 | 49 |
| | 61 | 2258 | 1082 | 48 |
| | 91 | 2057 | 935 | 46 |
| HTC (pmol/L) | 1 | 34.32 | 8.11 | 24 |
| | 15 | 899 | 353 | 39 |
| | 61 | 739 | 295 | 40 |
| | 91 | 877 | 405 | 46 |
| MMA (nmol/L) | 1 | 232 | 142 | 61 |
| | 15 | 128 | 41 | 32 |
| | 61 | 148 | 77 | 52 |
| | 91 | 149 | 63 | 42 |
| Homocysteine (µmol/L) | 1 | 13 | 3 | 24 |
| | 15 | 10 | 3 | 28 |
| | 61 | 11 | 3 | 26 |
| | 91 | 12 | 4 | 31 |

**Table 9 - Oral Treatment Group**

| Parameter | Day | Mean | Standard Deviation (SD) | Percent Co-variance (%CV) |
|---|---|---|---|---|
| Total B₁₂ (pg/mL) | 1 | 390 | 186 | 48 |
| | 15 | 1677 | 1018 | 61 |
| | 61 | 1828 | 1134 | 62 |
| | 91 | 1914 | 946 | 49 |
| HTC (pmol/L) | 1 | 40 | 16 | 41 |
| | 15 | 540 | 379 | 70 |
| | 61 | 519 | 337 | 65 |
| | 91 | 626 | 432 | 69 |
| MMA (nmol/L) | 1 | 220 | 123 | 56 |
| | 15 | 143 | 62 | 43 |
| | 61 | 142 | 88 | 62 |
| | 91 | 134 | 56 | 42 |
| Homocysteine (µmol/L) | 1 | 14 | 5 | 34 |
| | 15 | 11 | 3 | 27 |
| | 61 | 10 | 3 | 26 |
| | 91 | 11 | 3 | 29 |

### Safety Profile

A total of 50 healthy adult male and female subjects were exposed at least once to study drug during this study. Overall, 28 (56.0%) subjects reported at least one AE during the study. All AEs reported were considered treatment-emergent AEs (TEAEs). Thirteen (54.2%) and 15 (57.7%) subjects reported at least one AE following administration of oral B₁₂ and IM B₁₂, respectively. Three (11.5%) subjects, all receiving IM B₁₂, reported serious adverse events (SAEs). No subjects discontinued study treatment because of an AE.

An overview of adverse events reported during this study is presented in Table 10.

**Table 10**

| Category* | Treatment | | Overall (N=50) |
|---|---|---|---|
| | Oral B₁₂ (N=24) | IM B₁₂ (N=26) | |
| | n (%) | n (%) | n (%) |
| Subjects with AEs | 13 (54.2) | 15 (57.7) | 28 (56.0) |
| Subjects with TEAEs | 13 (54.2) | 15 (57.7) | 28 (56.0) |
| Subjects with SAEs | 0 (0) | 3 (11.5) | 3 (6.0) |
| Subjects who discontinued because of an AE | 0 (0) | 0 (0) | 0 (0) |

| | | | |
|---|---|---|---|
| * - Subjects may fall into more than one category. | | | |

No subject discontinued treatment due to an AE. Three subjects, all randomized to receive IM B₁₂, experienced a SAE during this study.

### Discussion:

The study demonstrated that both oral and IM B₁₂ treatments produced cobalamin normalization (>350 pg/ml) in all members of this population of mildly B₁₂ deficient subjects in 15 days or less. In addition this effect was maintained for the 3-month duration of the study. The study population included subjects who might have been expected to have age-related B₁₂ deficiency and some who had underlying gastrointestinal conditions known to interfere with B₁₂ absorption. Despite normalization of cobalamin in all subjects, differences were apparent between the two treatment regimens. The standard IM dosing regimen used in this study involved multiple injections in the first month and produced higher values for cobalamin than oral B₁₂. With IM dosing frequency changed to monthly, mean cobalamin values trended lower between months 2 and 3. In contrast daily oral B₁₂ treatment resulted in improvement in cobalamin values throughout the study and by Day 91 the mean values for the two treatment groups were similar (2057±935 pg/mL and 1914±946 pg/mL for IM and oral B₁₂, respectively). All patients tested to date in this study have responded positively to the SNAC / vitamin B₁₂ treatment.

The principles, preferred embodiments, and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art, without departing from the spirit of the invention.

## Claims

1. An oral pharmaceutical composition in the form of tablets comprising:
(i) 100 mg sodium N-[8-(2-hydroxybenzoyl)amino]caprylate;
(ii) 1 mg vitamin B12;
(iii) 2 mg povidone;
(iv) 95 mg dibasic calcium phosphate; and
(v) 2 mg magnesium stearate,
for use in the treatment of vitamin B12 deficiency defined as serum cobalamin below 350 pg/ml, wherein the composition is administered once daily.

## Patentansprüche

1. Orale pharmazeutische Zusammensetzung in Form von Tabletten, umfassend:
(i) 100 mg Natrium-N-[8-(2-hydroxybenzoyl)amino]caprylat;
(ii) 1 mg Vitamin B₁₂;
(iii) 2 mg Povidon;
(iv) 95 mg zweibasisches Calciumphosphat und
(v) 2 mg Magnesiumstearat,
zur Verwendung bei der Behandlung von Vitamin B12-Mangel, der durch Serum-Cobalamin unter 350 pg/ml definiert ist, wobei die Zusammensetzung einmal täglich verabreicht wird.

## Revendications

1. Composition pharmaceutique orale sous la forme de comprimés comprenant :
(i) 100 mg de sodium N-[8-(2-hydroxybenzoyl)amino]caprylate ;
(ii) 1 mg de vitamine B₁₂ ;
(iii) 2 mg de povidone ;
(iv) 95 mg de phosphate de calcium dibasique ; et
(v) 2 mg de stéarate de magnésium,
en vue de son utilisation dans le traitement de la carence en vitamine B12 définie comme cobalamine de sérum en-dessous de 350 pg/ml, dans laquelle la composition est administrée une fois par jour.
